# FASCICULE DE BREVET EUROPEEN

(11) **EP 4 076 270 B1**
(45) Date de publication et mention de la délivrance du brevet: **17.01.2024**
(21) Numéro de dépôt: 20845206.0
(22) Date de dépôt: 15.12.2020
(51) Int. Cl.: A61F 2/00

(54) **PROCEDE DE PURGE D'UN DISPOSITIF MEDICAL ADAPTE POUR ETRE IMPLANTE**
VERFAHREN ZUM ENTLEEREN EINER IMPLANTIERBAREN MEDIZINISCHEN VORRICHTUNG
METHOD FOR DRAINING A MEDICAL DEVICE ADAPTED TO BE IMPLANTED

(30) Priorité: 16.12.2019 FR 1914526
(43) Date de publication de la demande: 26.10.2022
(73) Titulaire: Uromems, 38320 Eybens (FR)
(72) Inventeur: LAMRAOUI, Hamid, 38410 Vaulnaveys le Haut (FR); MOZER, Pierre, 94300 Vincennes (FR); BEAUGERIE, Aurélien, 92130 Issy-les-Moulineaux (FR); MIR, Riaz, 38600 Fontaine (FR); HO, Thierry, 38120 Saint Egrève (FR)
(74) Mandataire: Regimbeau
(86) Numéro de dépôt international: PCT/FR2020/052446
(87) Numéro de publication internationale: WO 2021/123604

(56) Documents cités:
- WO-A1-2016/083428
- JP-A- S61 154 664
- US-A- 4 750 619

## Description

### Domaine technique

L'invention concerne un emballage pour un dispositif médical adapté pour être implanté dans un corps humain ou animal comprenant un réservoir de fluide.

### Etat de la technique

Divers dispositifs médicaux implantables comprennent un réservoir de fluide biocompatible couplé à un circuit fluidique. Un tel circuit fluidique peut par exemple permettre d'actionner un élément du dispositif.

Par exemple, un dispositif pour obturer sélectivement un conduit anatomique, tel que l'urètre ou le col vésical, peut comprendre une manchette occlusive entourant ledit conduit anatomique et couplée fluidiquement à un réservoir de fluide. Une pompe permet de transférer du fluide du réservoir vers la manchette, de sorte à augmenter la compression exercée par la manchette sur le conduit, ou de la manchette vers le réservoir, de sorte à réduire la compression exercée par la manchette sur le conduit.

D'autres applications impliquant un réservoir de fluide dans un dispositif médical implanté dans le corps d'un patient concernent le gonflage d'un ballon d'obturation, ou encore le remplissage d'une prothèse.

En général, le dispositif médical adapté pour être implanté est fourni au chirurgien vide de tout fluide biocompatible, dans un emballage dont le contenu est stérile. Le chirurgien doit donc chasser l'air et remplir le réservoir d'un fluide biocompatible avant l'implantation chez le patient.

Cette opération de remplissage doit être mise en oeuvre de manière à purger le circuit fluidique de l'air contenu initialement. En effet, d'éventuelles bulles d'air sont susceptibles d'affecter le fonctionnement du dispositif implantable et en particulier sa précision.

Une autre contrainte de l'opération de remplissage est le maintien de la stérilité du dispositif.

Un remplissage manuel du dispositif par le chirurgien allonge la durée de l'intervention chirurgicale et induit un risque de contamination du dispositif, susceptible d'induire des infections ou des complications chez le patient.

Il existe des dispositifs médicaux préremplis mais ces derniers présentent de nombreux désavantages comme par exemple lors de l'étape de stérilisation pendant laquelle il faut s'assurer que le fluide est également stérilisé à travers le dispositif.

Le document JP S61 154664 A divulgue un dispositif médical comprenant un boîtier renfermant un réservoir de fluide et une sortie fluidique formant une liaison fluidique entre le réservoir et un volume extérieur au boîtier.

### Brève description de l'invention

Un but de l'invention est donc de définir un procédé de purge d'un dispositif médical adapté pour être implanté comprenant un réservoir de fluide ne nécessitant pas de manipulation du dispositif.

A cet effet, un premier objet de l'invention concerne un procédé de purge d'un dispositif médical adapté pour être implanté dans un corps humain ou animal, ledit dispositif médical comprenant un boîtier renfermant un réservoir de fluide et une sortie fluidique formant une liaison fluidique entre le réservoir et un volume extérieur au boîtier, comprenant :
- la fourniture du dispositif dans un emballage comprenant une cuve, le boîtier étant maintenu dans la cuve dans une position dans laquelle le réservoir est positionné entre le fond de la cuve et la sortie fluidique,
- le remplissage de la cuve avec un fluide biocompatible, de sorte à immerger dans le fluide une zone de l'emballage extérieure au dispositif médical qui est en liaison fluidique avec la sortie fluidique du réservoir,
- la mise en oeuvre d'une purge du dispositif médical par remplissage du réservoir par le fluide biocompatible et évacuation de l'air présent du réservoir à la sortie fluidique.

De manière préférée, le fluide biocompatible peut être un liquide.

Le procédé peut comprendre une étape de vérification de l'horizontalité du fond de la cuve.

Selon un mode de réalisation, ladite vérification est basée sur un indicateur d'horizontalité du niveau de fluide dans la cuve.

Selon un autre mode de réalisation, éventuellement combiné au précédent, ladite vérification de l'horizontalité est réalisée au moyen d'un accéléromètre du dispositif médical.

De manière alternative ou complémentaire aux deux modes de réalisation précédents, la cuve est immergée dans un récipient rempli d'un fluide pour obtenir une horizontalité du fond de la cuve.

Dans certains modes de réalisation, la mise en oeuvre de la purge du dispositif médical est réalisée par déplacement d'une paroi mobile du réservoir pour faire varier un volume du réservoir.

Ledit déplacement de la paroi mobile du réservoir peut être effectué par un actionneur électromécanique agencé dans le boîtier.

Dans certains modes de réalisation, le boîtier comprend une unique sortie fluidique et une unique liaison fluidique entre le réservoir et ladite sortie fluidique.

Dans certains modes de réalisation, le dispositif médical est adapté pour commander une occlusion d'un conduit naturel du corps humain ou animal.

Dans certains modes de réalisation, le dispositif médical est choisi dans le groupe consistant en un sphincter artificiel, un muscle artificiel, un stimulateur électrique, un anneau gastrique, un neurostimulateur et un implant pénien.

### Brève description des dessins

D'autres caractéristiques et avantages de l'invention ressortiront de la description détaillée qui va suivre, en référence aux dessins annexés, sur lesquels :
- la figure 1 est une vue éclatée d'un emballage selon un mode de réalisation ;
- la figure 2 est une vue en perspective de la cuve ;
- la figure 3 est une vue en coupe de l'emballage assemblé de la figure 1 ;
- la figure 4 est une vue en perspective du dispositif médical dans son emballage ;
- la figure 5 est un synoptique d'un procédé de purge selon un mode de réalisation.

### Description détaillée de modes de réalisation

Le dispositif médical comprend un boîtier en un matériau biocompatible, par exemple du titane.

Le boîtier contient un réservoir de fluide biocompatible et est pourvu d'une sortie fluidique qui forme une liaison fluidique entre le réservoir et le milieu extérieur au dispositif médical. Ladite sortie fluidique peut notamment être connectée à une tubulure assurant une liaison fluidique avec un élément extérieur au boîtier.

Selon un mode de réalisation, le réservoir comprend une paroi mobile, mue par un actionneur, dont le déplacement permet de faire varier le volume du réservoir de manière contrôlée. Cette variation de volume permet de déplacer du fluide du réservoir vers la sortie fluidique ou de la sortie fluidique vers le réservoir.

De manière particulièrement avantageuse, l'actionneur est un actionneur électromécanique, qui est commandé par une unité de commande pour déplacer la paroi mobile d'une distance déterminée. L'unité de commande peut être agencée dans le boîtier. Le dispositif médical est ainsi autonome, en ce sens qu'il ne nécessite pas d'action du praticien pour remplir sa fonction.

Par ailleurs, le dispositif comprend avantageusement une unique sortie fluidique et un unique circuit fluidique entre le réservoir et la sortie fluidique.

Dans certaines applications, le dispositif médical est un sphincter artificiel, destiné à obturer sélectivement un conduit anatomique en exerçant un effort de compression sur ledit conduit. En particulier, le dispositif médical peut être un sphincter urinaire artificiel.

Dans un tel dispositif, la compression est exercée sur le conduit anatomique au moyen d'une manchette occlusive reliée à la sortie fluidique du boîtier par une tubulure. L'actionneur électromécanique, en déplaçant la paroi mobile, permet de transférer du fluide du réservoir vers la manchette ou de la manchette vers le réservoir, afin d'ajuster la compression exercée par la manchette.

Des exemples d'un tel dispositif médical sont décrits dans le document WO 2016/083428.

Un tel dispositif se distingue des sphincters artificiels comprenant une pompe actionnable manuellement par le patient, un ballon de régulation de pression et une manchette, par le fait que le dispositif a un fonctionnement autonome commandable, sans action manuelle répétitive du patient.

Dans d'autres applications, le dispositif médical peut être un muscle artificiel, un stimulateur électrique, un anneau gastrique, un neurostimulateur ou un implant pénien (liste non limitative).

Entre le site de fabrication du dispositif médical et le bloc opératoire dans lequel le dispositif médical est implanté chez un patient, le dispositif médical est agencé dans un emballage. Ledit emballage est adapté pour maintenir le dispositif médical stérile en le protégeant des contaminations extérieures et pour le protéger des chocs. Dans l'emballage, le dispositif médical est vide de fluide biocompatible, le remplissage du circuit fluidique n'étant réalisé qu'au bloc opératoire.

Comme décrit par la suite, ledit emballage est en outre adapté pour permettre la purge du circuit fluidique sans manipulation du dispositif médical par un praticien au bloc opératoire.

L'emballage comprend tout d'abord une cuve dans laquelle le dispositif médical est maintenu dans une position dite verticale, c'est-à-dire dans laquelle dans le réservoir est positionné entre le fond de la cuve et la sortie fluidique.

La cuve comprend un fond horizontal, une paroi latérale, une face supérieure opposée au fond selon la direction verticale.

La cuve comprend au moins une ouverture configurée pour permettre le remplissage de la cuve avec un fluide biocompatible.

La sortie fluidique du dispositif médical est située en-dessous de ladite ouverture, de sorte que, lors du remplissage de la cuve, l'ensemble du dispositif médical, incluant la sortie fluidique, est immergé dans le fluide.

Pour s'assurer que le remplissage de la cuve en fluide biocompatible est suffisant pour mettre en oeuvre le procédé de purge, la cuve peut avantageusement comporter un indicateur visuel de niveau de liquide disposé dans la partie haute de la cuve. Avantageusement, le volume de la cuve et la position de l'indicateur ont été choisis et dimensionnés pour permettre le remplissage du réservoir du dispositif.

Alternativement, la cuve peut comporter une cavité en partie supérieure destinée à recevoir un trop-plein de fluide biocompatible de sorte à réguler le débordement de ce fluide et éviter un remplissage excessif.

Le dispositif médical est maintenu dans la position verticale par un premier élément de maintien agencé sur le fond, la paroi latérale et/ou la face supérieure de la cuve.

Selon un mode de réalisation, ledit premier élément de maintien se présente sous la forme d'un logement dont les dimensions sont adaptées pour recevoir une partie du boîtier en exerçant un léger serrage pour le maintenir dans la position souhaitée, sans l'endommager.

De manière avantageuse, ledit logement est agencé dans le fond de la cuve.

Selon un mode de réalisation préféré, la cuve est réalisée par exemple par moulage ou formage d'un matériau plastique, et le logement est intégré au fond de la cuve par moulage ou formage. Le logement peut par exemple se présenter sous la forme de deux cloisons parallèles distantes de la largeur du boîtier.

Dans certains modes de réalisation, le dispositif médical peut être emballé avec une tubulure en liaison fluidique avec la sortie fluidique. Dans ce cas, la cuve comprend avantageusement un second élément de maintien agencé sur le fond et/ou la paroi latérale de la cuve pour maintenir une portion de ladite tubulure en-dessous de la sortie fluidique.

De manière avantageuse, ledit second élément de maintien se présente sous la forme d'un logement agencé dans le fond de la cuve, parallèlement au logement du boîtier. Ce logement peut par exemple se présenter sous la forme de deux cloisons parallèles distantes du diamètre de la tubulure.

Selon un mode de réalisation adapté, représenté sur les figures, la cuve présente une forme parallélépipédique comprenant une paroi latérale à quatre côtés et un fond, la face opposée au fond étant ouverte. Cependant, cette forme particulière n'est aucunement limitative.

Ainsi, selon d'autres modes de réalisation non illustrés, la cuve peut présenter toute autre forme, telle qu'une forme cylindrique par exemple, avec une paroi latérale cylindrique et un fond circulaire.

De manière particulièrement avantageuse, le fond de la cuve présente des dimensions sensiblement identiques selon deux directions orthogonales dans un plan horizontal, afin d'assurer la stabilité de la cuve, et éviter notamment tout renversement de la cuve lors de la purge du dispositif médical.

Selon certains modes de réalisation non illustrés, l'ouverture de la cuve peut ne pas être située sur la face supérieure de la cuve mais sur la paroi latérale de la cuve, de préférence dans une portion supérieure de ladite paroi de sorte à se trouver au-dessus de la sortie fluidique du dispositif médical.

Dans tous les cas, la cuve est dimensionnée et l'ouverture est agencée de sorte que, lorsque le fond de la cuve s'étend dans un plan horizontal, la cuve puisse être remplie de fluide biocompatible au moins jusqu'au niveau de la sortie fluidique du dispositif médical, de préférence au-dessus de ladite sortie. Ainsi, lors de la purge, l'ensemble du circuit fluidique s'étendant entre le réservoir et la sortie fluidique peut être rempli de fluide biocompatible et purgé de l'air présent initialement.

De manière avantageuse, l'emballage comprend en outre un couvercle amovible vis-à-vis de la cuve de sorte à obturer au moins partiellement l'ouverture. Le couvercle est de préférence emboîté sur la paroi latérale, et retenu sur celle-ci par friction ou par emboitement par exemple.

Selon un mode de réalisation préféré, le couvercle est configuré pour être solidarisé à une partie seulement de la paroi latérale, le couvercle présentant au moins une zone de préhension distante de la paroi latérale. Par exemple, le couvercle peut présenter un contour dont une partie coïncide avec la forme de l'ouverture définie par la paroi latérale et une partie est en retrait de la paroi latérale, pour former un passage pour les doigts du praticien facilitant la préhension du couvercle.

De manière particulièrement avantageuse, le couvercle présente un autre élément de maintien adapté pour maintenir le dispositif médical dans la position verticale. Cet élément de maintien peut se présenter sous la forme d'un logement agencé en regard du logement formé dans la cuve. Ainsi, le dispositif médical est maintenu dans la position verticale en deux endroits, ce qui améliore sa stabilité notamment pendant son transport.

Comme la cuve, le couvercle peut être réalisé par moulage ou formage d'un matériau plastique, et le logement est intégré à l'intérieur du couvercle par moulage ou formage. Le logement peut par exemple se présenter sous la forme de deux cloisons parallèles distantes de la largeur du boîtier.

Dans certains modes de réalisation, l'emballage comprend un bac destiné à contenir la cuve. Le bac permet de renforcer la protection du dispositif médical contre les chocs. Le bac forme également avantageusement une enceinte stérile pour la cuve, ce qui améliore la protection du dispositif médical contre les contaminants extérieurs. De préférence, lors de l'opération d'implantation, le bac peut être manipulé et ouvert en dehors du champ stérile du bloc opératoire de sorte à pouvoir en extraire la cuve qui est transmise dans le champ stérile. La cuve est ensuite ouverte et manipulée par le praticien dans le champ stérile. Par ailleurs, comme expliqué plus bas, le bac peut être utilisé dans le procédé de purge, en étant rempli de fluide biocompatible.

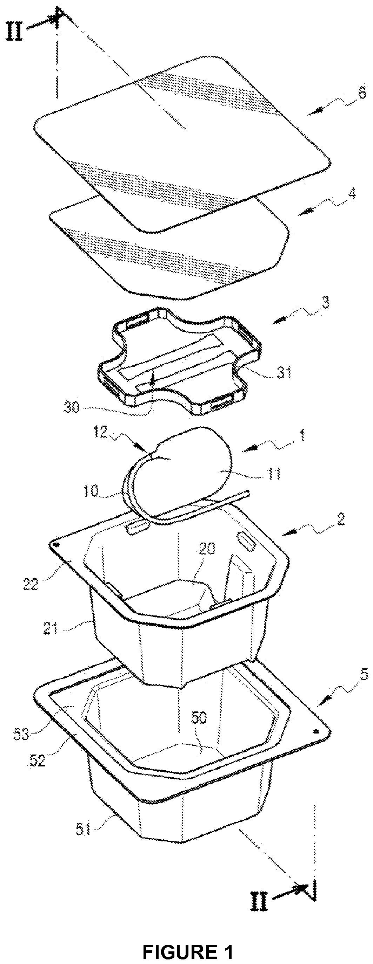

Le procédé de conditionnement du dispositif médical peut être mis en oeuvre de la façon suivante, avec l'emballage illustré sur la figure 1.

Le dispositif médical assemblé 1 est mis en place dans la cuve 2. A cet effet, on engage le dispositif médical dans le ou les élément(s) de maintien prévu(s) dans la cuve pour maintenir ledit dispositif avec la sortie fluidique au-dessus du réservoir. Lorsque la sortie fluidique est connectée à une tubulure, ladite tubulure est également de préférence engagée dans le ou les élément(s) de maintien prévu(s) dans la cuve pour maintenir ladite tubulure au fond de la cuve.

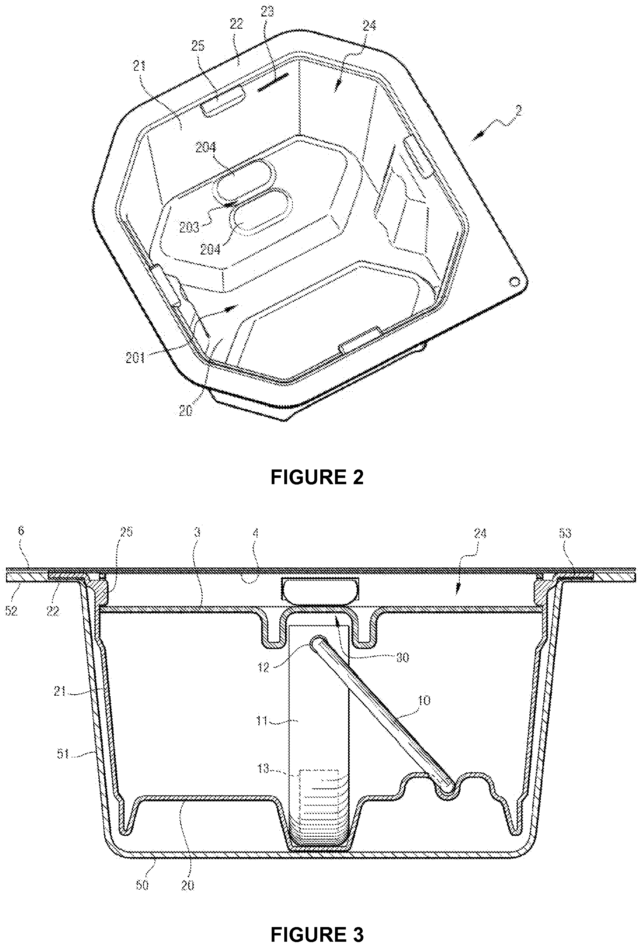

En référence à la figure 2, la cuve 2 comprend un fond 20 et une paroi latérale 21 qui s'étend sensiblement verticalement à partir du fond 20. Du côté opposé au fond 20, la paroi latérale 21 présente un bord 22 qui s'étend vers l'extérieur dans un plan horizontal. Ledit bord 22 définit une face supérieure de la cuve pourvue d'une ouverture 24.

Le dispositif médical 1 comprend un boîtier 11 dans lequel est agencé un réservoir de fluide biocompatible 13 (schématisé en pointillés sur la figure 3). Le dispositif médical 1 comprend en outre une sortie fluidique 12 qui est connectée au boîtier 11 et en liaison fluidique avec le réservoir 13. La sortie fluidique 12 assure une liaison fluidique entre le réservoir et l'extérieur du boîtier. La sortie fluidique 12 est connectée à une tubulure 10, qui est destinée à être reliée, lors de l'implantation, à la manchette occlusive.

La hauteur de la paroi latérale 21 est supérieure à la hauteur du dispositif médical dans sa position verticale de conditionnement. Ainsi, lorsque le dispositif médical 1 est agencé dans la cuve 2, comme illustré sur la figure 3, le dispositif médical est entièrement contenu dans la cuve 2. La sortie fluidique 12 est agencée sous l'ouverture 24, de sorte à pouvoir être recouverte de fluide lors du remplissage de la cuve. Le réservoir 13 est agencé sous la sortie fluidique 12 lorsque le dispositif média) est disposé dans la cuve.

Le fond 20 présente une forme sensiblement carrée afin d'assurer la stabilité de la cuve.

Le fond 20 de la cuve 2 comprend un logement 201 adapté pour maintenir le dispositif médical en position verticale. Le logement 201 est défini comme une cavité formée dans le fond 20, parallèlement à l'un des côtés, et s'étend sensiblement entre le milieu d'un côté et le milieu du côté opposé du fond 20. L'emplacement du logement est dans une région centrale du fond pour assurer la stabilité de l'ensemble cuve - dispositif médical lorsque le dispositif médical est agencé dans le logement et la cuve est remplie de fluide biocompatible. La largeur dudit logement est sensiblement égale à celle du dispositif médical, afin de permettre l'insertion et le maintien du dispositif médical dans ledit logement. La profondeur du logement est choisie de sorte à éviter tout basculement du boîtier 11 ; une profondeur de 5 à 15 mm est adaptée à cette fonction.

De manière particulièrement avantageuse, le logement 201 est pourvu de deux paires de protrusions arrondies (non illustrées), dans lesquelles les protrusions de chaque paire sont agencées en vis-à-vis dans le sens de la largeur du logement 201. Chaque paire de protrusions restreint localement la largeur du logement 201 et exerce ainsi un léger serrage sur le dispositif médical pour son maintien en position.

Le fond 20 de la cuve comprend en outre un logement 203 pour la tubulure 10 du dispositif médical. La largeur dudit logement est sensiblement égale au diamètre de la tubulure. Ledit logement se présente sous la forme de deux plots 204 parallèles en relief par rapport au fond 20. De manière avantageuse, comme pour le logement 201, les plots présentent une paire de protrusions arrondies (non illustrées) en vis-à-vis dans le sens de la largeur du logement 203. Ladite paire de protrusions restreint localement la largeur du logement 203 et exerce ainsi un léger serrage sur la tubulure. Le logement 203 est avantageusement parallèle au logement 201.

De manière alternative, le logement 201 pourrait être défini par une ou plusieurs paires de plots en vis-à-vis, en relief par rapport au fond de la cuve. Le logement 203 pourrait quant à lui être défini comme une cavité dans le fond de la cuve. Toute autre forme adaptée à un maintien du dispositif médical en position verticale pourrait être utilisée.

Le couvercle 3 est mis en place sur la cuve 2. Le dispositif médical est alors engagé dans le ou le(s) élément(s) de maintien prévu(s) dans le couvercle pour maintenir ledit dispositif avec la sortie fluidique au-dessus du réservoir.

Par exemple, le couvercle peut comprendre, en regard du logement 201, un logement 30 formé dans le couvercle. Le logement 30 peut être défini par deux plots en relief par rapport à la surface intérieure du couvercle, ou comme un creux formé dans la surface intérieure du couvercle. La largeur du logement 30 est sensiblement égale à la largeur de la partie supérieure du dispositif médical 1 dans la position verticale.

De manière avantageuse, le couvercle 3 présente sensiblement une forme de croix. De ce fait, le couvercle n'obture qu'une partie de l'ouverture supérieure de la cuve 2, et laisse au niveau des coins de la cuve des ouvertures adaptées pour la préhension du couvercle au niveau de zones de préhension 31 par un praticien.

Les formes et dimensions respectives de la paroi latérale 21 et du bord du couvercle 3 sont choisies pour permettre un emboîtement du couvercle dans la cuve. En particulier, la paroi latérale 21 peut comprendre, dans sa partie supérieure, un rebord 25 adapté pour retenir un bord respectif du couvercle 3.

Une première feuille 4 perméable à un gaz stérilisant, par exemple un opercule en Tyvek^{™}, est ensuite scellée sur le bord supérieur 22 de la cuve.

La cuve 2 est ensuite mise en place dans le bac 5.

Le bac 5 présente une forme similaire à celle de la cuve 2 mais des dimensions supérieures. De préférence, le bac 5 présente une taille suffisante pour recevoir entièrement la cuve 2 tout en minimisant l'espace libre entre la cuve 2 et le bac 5, afin de minimiser l'encombrement de l'emballage.

Le bac 5 présente un fond 50 sensiblement plan et une paroi latérale qui s'étend sensiblement verticalement à partir du fond 50. Du côté opposé au fond 50, la paroi latérale 51 présente un bord 52 qui s'étend vers l'extérieur dans un plan horizontal. Ledit bord 52 définit une face supérieure du bac pourvue d'une ouverture centrale.

De manière avantageuse, le bac 5 présente un rebord intérieur 53 procurant un appui pour le bord 22 de la cuve 2.

Comme le fond 20 de la cuve, le fond 50 du bac 5 est sensiblement carré pour assurer une bonne stabilité du bac.

Une seconde feuille 6 perméable à un gaz stérilisant, par exemple un opercule en Tyvek^{™}, est ensuite scellée sur le bord supérieur 52 du bac 5.

L'emballage ainsi scellé est stérilisé par tout moyen adapté, puis conditionné dans une boîte (non illustrée) qui constitue l'enveloppe externe dans laquelle le dispositif médical est livré à un praticien pour l'implantation chez un patient.

En vue de cette implantation, le praticien sort l'emballage de la boîte, retire la seconde feuille de scellage, sort la cuve puis retire la première feuille de scellage.

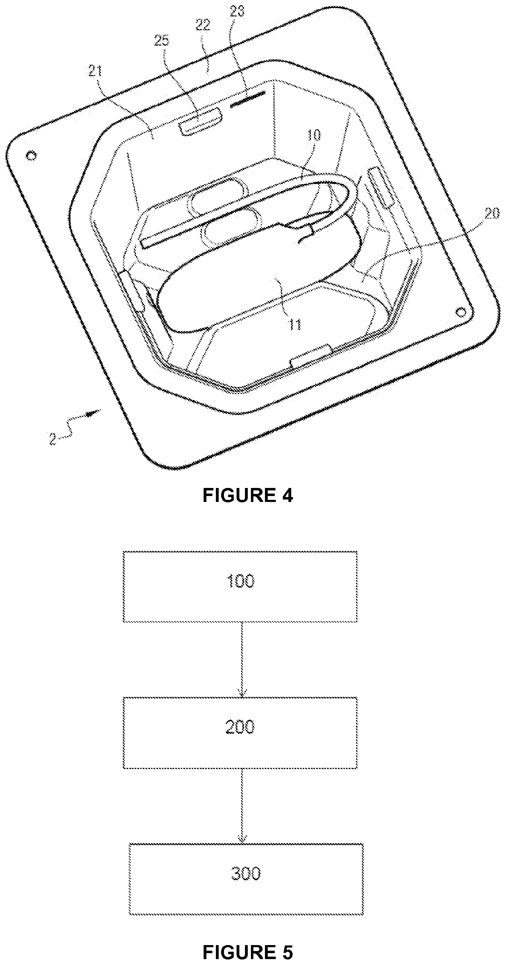

D'une manière générale, le procédé de purge du dispositif médical comprend les étapes suivantes, dont l'enchaînement est illustré sur la figure 5 :
- dans une étape 100, le praticien dispose du dispositif agencé dans la cuve, le boîtier étant maintenu dans la cuve dans la position verticale dans laquelle il a été emballé, comme illustré sur la figure 4 ; le réservoir est donc positionné entre le fond de la cuve et la sortie fluidique,
- dans une étape 200, le praticien remplit la cuve avec un fluide biocompatible, de sorte à immerger dans le fluide une zone de l'emballage extérieure au dispositif médical qui est en liaison fluidique avec la sortie fluidique du réservoir,
- dans une étape 300, la purge du dispositif médical est réalisée, permettant le remplissage du réservoir par le fluide biocompatible et l'extraction de tout l'air présent initialement dans le circuit fluidique.

Ces différentes étapes sont décrites plus en détail ci-après.

Selon un mode de réalisation, le praticien sort la cuve du bac en vue de la remplir de fluide biocompatible.

Pour effectuer ce remplissage et la mise en oeuvre de la purge, le praticien doit s'assurer que le fond de la cuve soit horizontal.

L'horizontalité du fond de la cuve peut être assurée en posant le fond de la cuve sur une surface plane et horizontale, par exemple une table.

Cette horizontalité peut être confirmée au moyen d'un indicateur adéquat. Par exemple, un tel indicateur d'horizontalité peut être un indicateur de l'horizontalité d'un niveau d'eau dans la cuve. Cet indicateur peut par exemple se présenter sous la forme d'un ou plusieurs segments formés dans la cuve, par exemple directement par moulage, voire par impression, et placés dans la partie supérieure de la cuve, parallèlement au fond. Un tel indicateur peut également servir d'indicateur de remplissage pour le praticien, lui signalant jusqu'à quelle hauteur de la cuve il doit verser le fluide biocompatible pour permettre une purge efficace. La figure 2 illustre un mode de réalisation d'un tel indicateur d'horizontalité sous la forme d'une ligne intérieure 23 en creux ou en relief s'étendant dans un plan horizontal à l'intérieur de la paroi latérale 21. De préférence, ladite ligne intérieure 23 s'étend sur plusieurs côtés de la paroi latérale 21, de manière continue ou discontinue.

Une indication sur l'horizontalité du fond de la cuve peut également être fournie par le dispositif médical lui-même. En effet, le dispositif comprend généralement un accéléromètre agencé dans le boîtier, qui peut être utilisé, lorsque le dispositif médical est implanté, pour déterminer une posture ou une activité du patient. Le boîtier étant solidaire du fond de la cuve avec une orientation déterminée par rapport à celui-ci (généralement perpendiculaire), l'accéléromètre peut fournir une indication de l'inclinaison du fond de la cuve.

De manière alternative ou complémentaire, cette horizontalité peut être assurée en plaçant la cuve dans un récipient rempli lui-même d'un fluide. Dans ce cas, la cuve est dimensionnée pour que, lorsqu'elle contient le dispositif médical dans la position prévue pour la purge, elle flotte dans le récipient en maintenant la sortie fluidique du dispositif médical au-dessus du réservoir.

Le bac dans lequel la cuve est agencée dans l'emballage peut être utilisé en tant que récipient à cet effet.

Le praticien remplit alors la cuve avec du fluide biocompatible qui est destiné au fonctionnement du dispositif médical. Ledit fluide peut être par exemple du sérum physiologique.

Il active ensuite l'actionneur du dispositif médical pour chasser l'air présent dans le circuit fluidique s'étendant entre le réservoir et la sortie fluidique et dans la tubulure le cas échéant, et remplir ledit circuit fluidique et la tubulure avec le fluide biocompatible. L'actionneur peut être activé plusieurs fois, par exemple trois fois, entrainant le déplacement d'une paroi mobile du réservoir pour faire varier un volume du réservoir et ainsi exercer une action de pompage, jusqu'à ce que le circuit fluidique soit entièrement purgé (aucune bulle d'air ne s'échappant de la sortie fluidique).

Cette activation de l'actionneur ne nécessite pas de manipulation du dispositif médical lui-même. En effet, le dispositif médical comprend des moyens de communication sans fil avec une télécommande externe, qui permet de paramétrer le dispositif et de lui envoyer des ordres d'activation ou de désactivation.

Une fois la purge achevée, le praticien peut obturer la sortie fluidique ou l'extrémité de la tubulure le cas échéant, et sort le dispositif médical de la cuve en vue de l'opération d'implantation.

Ainsi, avant l'implantation, le praticien n'a pas touché le dispositif médical, ce qui permet d'éviter ou tout au moins de minimiser les risques de contamination du dispositif.

Par ailleurs, l'intervention du praticien n'est pas requise pendant la mise en oeuvre de la purge. En effet, une fois que la purge a été enclenchée, c'est l'actionneur du dispositif qui aspire le fluide biocompatible et chasse l'air. Le praticien peut donc se consacrer à d'autres tâches, telles que la préparation de l'implantation. Le temps chirurgical peut ainsi être réduit de manière significative.

Enfin, le fait que la mise en oeuvre de la purge soit automatique accroit la fiabilité du procédé.

Il est entendu que les modes de réalisation décrits ci-dessus correspondent à des exemples particuliers et non-limitatifs et que diverses modifications peuvent être apportées sans pour autant s'éloigner de l'invention.

## Revendications

1. Procédé de purge d'un dispositif médical (1) adapté pour être implanté dans un corps humain ou animal, ledit dispositif médical comprenant un boîtier (11) renfermant un réservoir de fluide (13) et une sortie fluidique (12) formant une liaison fluidique entre le réservoir et un volume extérieur au boîtier, comprenant :
- la fourniture du dispositif dans un emballage comprenant une cuve (2), le boîtier étant maintenu dans la cuve dans une position dans laquelle le réservoir est positionné entre le fond de la cuve et la sortie fluidique,
- le remplissage de la cuve (2) avec un fluide biocompatible, de sorte à immerger dans le fluide une zone de l'emballage extérieure au dispositif médical qui est en liaison fluidique avec la sortie fluidique du réservoir,
- la mise en oeuvre d'une purge du dispositif médical par remplissage du réservoir (13) par le fluide biocompatible et évacuation de l'air présent du réservoir (13) à la sortie fluidique (12).

2. Procédé selon la revendication 1, comprenant une étape de vérification de l'horizontalité du fond (20) de la cuve.

3. Procédé selon la revendication 2, dans lequel ladite vérification est basée sur un indicateur d'horizontalité du niveau de fluide dans la cuve (2).

4. Procédé selon l'une des revendications 2 ou 3, dans lequel ladite vérification de l'horizontalité est réalisée au moyen d'un accéléromètre du dispositif médical.

5. Procédé selon l'une des revendications 2 à 4, dans lequel la cuve est immergée dans un récipient (5) rempli d'un fluide pour obtenir une horizontalité du fond de la cuve.

6. Procédé selon l'une des revendications 1 à 5, dans lequel l'étape de la mise en oeuvre d'une purge du dispositif médical est réalisée par déplacement d'une paroi mobile du réservoir pour faire varier un volume du réservoir.

7. Procédé selon la revendication 6, dans lequel ledit déplacement de la paroi mobile du réservoir est effectué par un actionneur électromécanique agencé dans le boîtier.

8. Procédé selon l'une des revendications 1 à 6, dans lequel le boîtier (11) comprend une unique sortie fluidique (12) et une unique liaison fluidique entre le réservoir et ladite sortie fluidique.

9. Procédé selon l'une des revendications 1 à 8, dans lequel le dispositif médical (1) est adapté pour commander une occlusion d'un conduit naturel du corps humain ou animal.

10. Procédé selon l'une des revendications 1 à 9, dans lequel le dispositif médical (1) est choisi dans le groupe consistant en un sphincter artificiel, un muscle artificiel, un stimulateur électrique, un anneau gastrique, un neurostimulateur et un implant pénien.

## Patentansprüche

1. Verfahren zum Entleeren einer medizinischen Vorrichtung (1), die dazu ausgelegt ist, in einen menschlichen oder tierischen Körper implantiert zu werden, wobei die medizinische Vorrichtung ein Gehäuse (11), das ein Fluidreservoir (13) umschließt, und einen Fluidauslass (12) umfasst, der eine Fluidverbindung zwischen dem Reservoir und einem Volumen außerhalb des Gehäuses bildet, umfassend:
- Bereitstellen der Vorrichtung in einer Verpackung, die einen Behälter (2) umfasst, wobei das Gehäuse im Behälter in einer Position gehalten wird, in der das Reservoir zwischen dem Boden des Behälters und dem Fluidauslass positioniert ist,
- Füllen des Behälters (2) mit einem biokompatiblen Fluid, so dass ein Bereich der Verpackung außerhalb der medizinischen Vorrichtung, der in Fluidverbindung mit dem Fluidauslass des Reservoirs steht, in das Fluid eingetaucht wird,
- Entleeren der medizinischen Vorrichtung durch Füllen des Reservoirs (13) mit dem biokompatiblen Fluid und Ablassen der im Reservoir (13) vorhandenen Luft zum Fluidauslass (12).

2. Verfahren nach Anspruch 1, umfassend einen Schritt des Überprüfens der Horizontalität des Bodens (20) des Behälters.

3. Verfahren nach Anspruch 2, wobei das Überprüfen auf einem Indikator der Horizontalität des Fluidstands im Behälter (2) basiert.

4. Verfahren nach einem der Ansprüche 2 oder 3, wobei das Überprüfen der Horizontalität mit Hilfe eines Beschleunigungsmessers der medizinischen Vorrichtung durchgeführt wird.

5. Verfahren nach einem der Ansprüche 2 bis 4, wobei der Behälter in ein mit einem Fluid gefülltes Gefäß (5) eingetaucht wird, um eine Horizontalität des Bodens des Behälters zu erreichen.

6. Verfahren nach einem der Ansprüche 1 bis 5, wobei der Schritt des Entleerens der medizinischen Vorrichtung durch Bewegen einer beweglichen Wand des Behälters durchgeführt wird, um ein Volumen des Reservoirs zu variieren.

7. Verfahren nach Anspruch 6, wobei das Bewegen der beweglichen Wand des Reservoirs durch einen im Gehäuse angeordneten elektromechanischen Aktuator ausgeführt wird.

8. Verfahren nach einem der Ansprüche 1 bis 6, wobei das Gehäuse (11) einen einzigen Fluidauslass (12) und eine einzige Fluidverbindung zwischen dem Reservoir und dem Fluidauslass umfasst.

9. Verfahren nach einem der Ansprüche 1 bis 8, wobei die medizinische Vorrichtung dazu ausgelegt ist, einen Verschluss eines natürlichen Trakts des menschlichen oder tierischen Körpers zu kontrollieren.

10. Verfahren nach einem der Ansprüche 1 bis 9, wobei die medizinische Vorrichtung (1) aus der Gruppe bestehend aus einem künstlichen Schließmuskel, einem künstlichen Muskel, einem Elektrostimulator, einem Magenband, einem Neurostimulator und einem Penisimplantat ausgewählt ist.

## Claims

1. A method for draining a medical device (1) adapted to be implanted in a human or animal body, said medical device comprising a casing (11) enclosing a fluid reservoir (13) and a fluid outlet (12) forming a fluid connection between the reservoir and a volume external to the casing, comprising:
- providing the device in a packaging comprising a tank (2), the casing being held in the tank in a position in which the reservoir is positioned between the bottom of the tank and the fluid outlet,
- filling the tank (2) with a biocompatible fluid, so that a zone of the packaging external to the medical device which is in fluid connection with the fluid outlet of the reservoir is immersed in the fluid,
- draining the medical device by filling the reservoir (13) with the biocompatible fluid and discharging the air present in the reservoir (13) to the fluid outlet (12).

2. The method according to claim 1, comprising a step of checking horizontality of the bottom (20) of the tank.

3. The method according to claim 2, wherein said checking is based on a horizontality indicator of the fluid level in the tank (2).

4. The method according to one of claims 2 or 3, wherein said checking of horizontality is performed by means of an accelerometer of the medical device.

5. The method according to one of claims 2 to 4, wherein the tank is immersed in a container (5) filled with a fluid to obtain horizontality of the bottom of the tank.

6. The method according to one of claims 1 to 5, wherein the step of draining the medical device is performed by moving a movable wall of the tank to vary a volume of the reservoir.

7. The method according to claim 6, wherein said movement of the movable wall of the reservoir is performed by an electromechanical actuator arranged in the casing.

8. The method according to one of claims 1 to 6, wherein the casing 11) comprises a single fluid outlet (12) and a single fluid connection between the reservoir and said fluid outlet.

9. The method according to one of claims 1 to 8, wherein the medical device (1) is adapted to control an occlusion of a natural duct of the human or animal body.

10. The method according to one of claims 1 to 9, wherein the medical device (1) is selected from the group consisting of an artificial sphincter, an artificial muscle, an electrical stimulator, a gastric band, a neurostimulator, and a penile implant.
